# EUROPÄISCHE PATENTSCHRIFT

(11) **EP 2 835 151 B1**
(45) Veröffentlichungstag und Bekanntmachung des Hinweises auf die Patenterteilung: **13.12.2017**
(21) Anmeldenummer: 14178853.9
(22) Anmeldetag: 29.07.2014
(51) Int. Cl.: A61Q 3/00, A61K 8/64, A61Q 5/12

(54) **Methionyl-Methionin-Stereoisomere und deren Verwendung in Kosmetika**
Methionyl-methionine stereoisomers and use thereof in cosmetics
Stéréo-isomère méthionyl-méthionine et son utilisation en cosmétique

(30) Priorität: 06.08.2013 DE 102013215434
(43) Veröffentlichungstag der Anmeldung: 11.02.2015
(73) Patentinhaber: Evonik Degussa GmbH, 45128 Essen (DE)
(72) Erfinder: Farwick, Mike, 45138 Essen (DE); Mentel, Matthias, 44357 Dortmund (DE); Maczkiewitz, Ursula, 45219 Essen (DE); Seifert, Andreas, 46238 Bottrop (DE)

(56) Entgegenhaltungen:
- WO-A1-2007/068401
- DE-A1-102008 042 932

## Beschreibung

### Gebiet der Erfindung

Gegenstand der Erfindung sind kosmetischen Formulierungen enthaltend bestimmte Methionyl-Methionin-Stereoisomere und die Verwendung von Methionyl-Methionin zur Nagel- und/oder Haarbehandlung.

### Stand der Technik

Methionin ist ein gebräuchlicher Wirkstoff zur Verbesserung der Eigenschaften von Haaren. So wird die Verwendung von Haarpflegezusammensetzungen enthaltend Methionin zur Verbesserung der Stärke und des Zustandes der Haare beispielsweise in der WO00/51556 beschrieben.

Die kosmetische Verwendung von Dipeptiden enthaltend mindestens ein Methionin der Formel XM oder MX, insbesondere L-Methionyl-L-Methionin, zur Hautaufhellung wird in der JP2999301 beschrieben.

Die Produktion und der Einsatz von stereoisomeren Mischungen von Methionyl-Methionin und seinen Salzen als Futtermitteladditiv für Fische und Krustentiere wird in der WO2010/043558 beschrieben. Bevorzugt liegt das beschriebene Dipeptid in der Futtermittelmischung als DD/LL/LD/DL-Mischung, als DL/LD- oder DD/LL-Mischung vor, und in einer besonders bevorzugten Verwendung liegt das Dipeptid als Enantiomerenpaar D-Methionyl-L-Methionin und L-Methionyl-D-Methionin vor. WO 2007/068401 offenbart die Verwendung von Dipeptiden für die Behandlung geschädigter Haare. Aufgabe der Erfindung war es, einen Ersatzstoff für Methionin in Kosmetika bereitzustellen.

### Beschreibung der Erfindung

Überraschenderweise wurde gefunden, dass die im Folgenden beschriebenen kosmetischen Formulierungen enthaltend bestimmte Methionyl-Methionin-Stereoisomere die oben genannte Aufgabe zu lösen vermögen. Überraschend und unerwartet wurde gefunden, dass diese Stereoisomere über herausragende und sehr breite Anwendungseigenschaften verfügen. Diese Eigenschaften stellen in bestimmten Anwendungen die von L-Methionin in den Schatten. Sie übertreffen außerdem in der haarspezifischen Anwendungstechnik die Eigenschaften von anderen gängigen Marktprodukte wie z.B. von hydrolysierten Weizenproteinen.

Gegenstand der vorliegenden Erfindung ist daher eine kosmetische Formulierung enthaltend bestimmte Methionyl-Methionin-Stereoisomere.
Ein weiterer Gegenstand der Erfindung ist die Verwendung von Methionyl-Methionin zur Nagel- und/oder Haarbehandlung.

Vorteil der vorliegenden Erfindung ist es, dass die Methionyl-Methionin-Stereoisomere eine niedrige Zytotoxizität aufweisen,
Ein weiterer Vorteil der vorliegenden Erfindung ist es, dass die Methionyl-Methionin-Stereoisomere eine gute Stabilität in wässriger Lösung aufweisen.
Noch ein Vorteil der vorliegenden Erfindung ist es, dass Methionyl-Methionin-Stereoisomere einen relativ geringen Geruch aufweisen.
Noch ein Vorteil der vorliegenden Erfindung, dass die Methionyl-Methionin in der Lage ist, sowohl Eigenschaften wie Kämmbarkeit, Weichheit, Volumen, Formbarkeit, Handhabbarkeit, die Entwirrbarkeit von ungeschädigten und geschädigten Haaren zu verbessern, als auch dem Haar einen schönen Glanz zu verleihen.

Von dem Dipeptid Methionyl-Methionin der allgemeinen Formel (I) 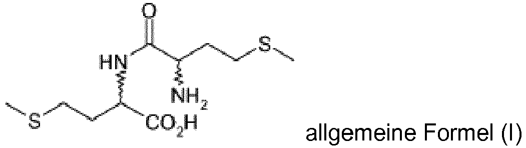 existieren vier verschiedene Stereoisomere: D-Methionyl-L-Methionin, L-Methionyl-D-Methionin, L-Methionyl-L-Methionin und D-Methionyl-D-Methionin:

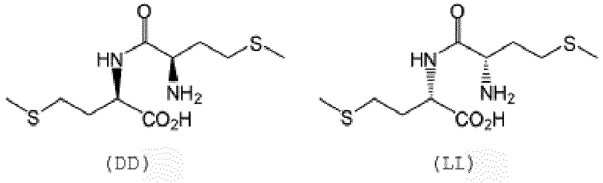

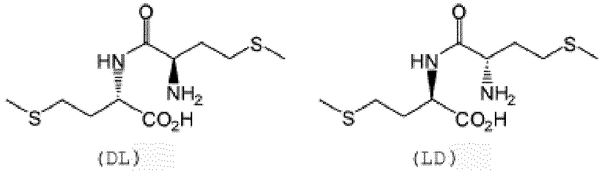

Dabei verhalten sich DD und LL untereinander wie Bild und Spiegelbild, d.h. es sind Enantiomere und besitzen damit auch die gleichen physikalischen Eigenschaften. Analoges gilt für das Paar DL und LD.

Die beiden Paare DD/LL und DL/LD sind dagegen diastereomer zueinander, d.h. sie haben unterschiedliche physikalische Daten. So besitzt beispielsweise das Enantiomerenpaar DD/LL bei Raumtemperatur eine Löslichkeit von 21 g/L in Wasser, wohingegen die Löslichkeit des Enantiomerenpaars DL/LD bei 0,4 g/L liegt.

Gegenstand der vorliegenden Erfindung ist daher eine kosmetische Formulierung enthaltend mindestens ein Methionyl-Methionin-Stereoisomer ausgewählt aus der Gruppe
D-Methionyl-L-Methionin, L-Methionyl-D-Methionin und D-Methionyl-D-Methionin, dadurch gekennzeichnet, dass sie ein Gewichtverhältnis von DL- und LD-Methionyl-Methionin zu DD- und LL-Methionyl-Methionin von 9 zu 1 bis 3 zu 2 aufweist. Der Begriff "D-Methionyl-L-Methionin" hat im Zusammenhang mit der vorliegenden Erfindung dieselbe Bedeutung wie DL-Methionyl-Methionin". Analoges gilt für L-Methionyl-D-Methionin, L-Methionyl-L-Methionin und D-Methionyl-D-Methionin Die erfindungsgemäße Verwendung ist in dem Falle, dass es sich um eine Verwendung an einer lebenden Spezies handelt, ausschließlich eine kosmetische und eine nicht-therapeutische Verwendung.

Alle angegebenen Prozent (%) sind, wenn nicht anders angegeben, Massenprozent. Bevorzugt sind in der erfindungsgemäßen Formulierung alle Methionyl-Methionin-Stereoisomere ausgewählt aus der Gruppe D-Methionyl-L-Methionin, L-Methionyl-D-Methionin und D-Methionyl-D-Methionin enthalten.

Es kann erfindungsgemäß vorteilhaft sein, wenn die erfindungsgemäße Formulierung zusätzlich L-Methionyl-L-Methionin enthält, weil beispielsweise die Anwesenheit von L-Methionyl-L-Methionin durch das Produktionsverfahren der Stereoisomere bedingt ist. Bevorzugt sind in der erfindungsgemäßen Formulierung somit alle Methionyl-Methionin-Stereoisomere ausgewählt aus der Gruppe
D-Methionyl-L-Methionin, L-Methionyl-D-Methionin, L-Methionyl-L-Methionin und D-Methionyl-D-Methionin
enthalten.

Bevorzugt beträgt die Gesamtmenge an L-Methionyl-L-Methionin bezogen auf das Gesamtgewicht aller in der erfindungsgemäßen Formulierung enthaltenen Methionyl-Methionin-Stereoisomere weniger als oder gleich 50 Gew.-%, bevorzugt weniger als 35 Gew.-%, besonders bevorzugt weniger als 20 Gew.-%.

Erfindungsgemäß bevorzugte Formulierungen weisen ein Gewichtverhältnis von DL- und LD-Methionyl-Methionin zu DD- und LL-Methionyl-Methionin von bevorzugt 8 zu 2 insbesondere 7 zu 3 bis 3 zu 2 auf, wobei je Enantiomerenpaar jeweils die Summe der einzelnen Enantiomere berücksichtigt wird. Die vorgenannte Formulierung schließt nicht aus, dass in der Formulierung nur eines der Enantiomere eines Enantiomerenpaares enthalten ist.

In einer alternativen bevorzugten Verwendung liegt DL- und LD-Methionyl-Methionin zu DD- und LL-Methionyl-Methionin im Verhältnis 10:90-0,01:99,99 vor.

Die erfindungsgemäßen Formulierungen können z.B. mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
I nsektrepellentien,
Selbstbräuner,
Konservierungsstoffe,
Konditioniermittel,
Parfüme,
Farbstoffe,
Geruchsabsorber,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.
Ein weiterer Gegenstand der vorliegenden Erfindung ist die Verwendung von Methionyl-Methionin zur Konditionierung von Haaren, zur Erhöhung der Zugfestigkeit der Haare, zur Erhöhung der Belastbarkeit von Haaren, zum Schutz der Haare vor UV-Schädigung, zum Schutz der Haare vor oxidativer Schädigung, zum Schutz der Haare vor thermischer Schädigung, zum Schutz der Haare vor chemischer Schädigung, wie zum Beispiel durch alkalische Behandlung oder durch Behandlung mit Reduktionsmitteln, und/oder zur Verbesserung der mechanischen Eigenschaften von Nägeln.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:
Abbildungen 1. Auswaschbarkeit von Methionyl-Methionin-Stereoisomeren nach Konditioniererbehandlung über 30 Minuten
Abbildungen 2. Auswaschbarkeit von Methionyl-Methionin-Stereoisomeren nach Konditioniererbehandlung über 24 Stunden.
Abbildung 3. Reparatur von gebleichtem Haar. Dargestellt sind die Verbesserungen nach Haarbehandlung mit einfachem Haarkonditionierer (Tabelle 3) des mittels Tensile Tester bestimmten Parameters Load bei 15%iger Dehnung. Dargestellte Werte sind in Millinewton (mN).
Abbildung 4. Reparatur von gebleichtem Haar. Dargestellt sind die Verbesserungen nach Haarbehandlung mit einfachem Haarkonditionierer (Tabelle 3) des mittels Tensile Tester bestimmten Parameters Work bis 15%iger Dehnung. Dargestellte Werte sind in Mikrojoule (µJ).
Abbildung 5. Reparatur von gebleichtem Haar. Dargestellt sind die Verbesserungen nach Haarbehandlung mit erweitertem Haarkonditionierer (Tabelle 4) des mittels Tensile Tester bestimmten Parameters Load bei 15%iger Dehnung. Dargestellte Werte sind in Millinewton (mN).
Abbildung 6. Reparatur von gebleichtem Haar. Dargestellt sind die Verbesserungen nach Haarbehandlung mit erweitertem Haarkonditionierer (Tabelle 4) des mittels Tensile Tester bestimmten Parameters Work bis 15%iger Dehnung. Dargestellte Werte sind in Mikrojoule (µJ).
Abbildung 7. Reparatur von gebleichtem Haar. Dargestellt sind die Verbesserungen nach Haarbehandlung mit Shampoo (Tabelle 5) des mittels Tensile Tester bestimmten Parameters Load bei 15%iger Dehnung. Dargestellte Werte sind in Millinewton (mN). Abbildung 8. Reparatur von gebleichtem Haar. Dargestellt ist die mittels Automatic Cyclic Tester bestimmte charakteristische Lebensdauer α nach Haarbehandlung mit einfachem Haarkonditionierer (Tabelle 3). Dargestellte Werte sind Anzahl der Zyklen, bei der 63.2% aller Haarfasern reißen.
Abbildung 9. Reparatur von alkalisch geglättetem Haar. Dargestellt sind die Verbesserungen nach Haarbehandlung mit einfachem Haarkonditionierer (Tabelle 8) des mittels Tensile Tester bestimmten Parameters Load bei 15%iger Dehnung. Dargestellte Werte sind in Millinewton (mN).
Abbildung 10. Reparatur von reduktiv geglättetem Haar. Dargestellt sind die Verbesserungen nach Haarbehandlung mit einfachem Haarkonditionierer (Tabelle 9) relativ zu Vehikel des mittels Tensile Tester bestimmten Parameters Load bei 15%iger Dehnung. Dargestellte Werte sind in Millinewton (mN).
Abbildung 11. Schutz des Haares vor thermischem Stress (Glätteisenbehandlung) durch präventive Behandlung mit Wirkstofflösung. Dargestellt ist die mittels Automatic Cyclic Tester bestimmte charakteristische Lebensdauer α bei 33%, 20% und 10% Überlebenswahrscheinlichkeit.
Abbildung 12. Schutz des Haares vor thermischem Stress (Glätteisenbehandlung) durch präventive Schutzbehandlung mit Wirkstofflösung. Dargestellt ist die Verbesserung der durch differentielle Scanning-Kalorimetrie bestimmten Haardenaturierungstemperatur.

### Beispiele:

### Beispiel 1: Verbessertes Auswaschverhalten durch Behandlung mit Methionyl-Methionin-Stereoisomeren

Tressenteile menschlicher Euro-Natur-Haare, remis, doppelt-gezogen mit einer Länge von 23 cm und einer Breite von 2.0 cm mit einem Gewicht von 2.0 g und dunkelbrauner Farbe wurden unter dem laufenden Wasserhahn innerhalb von 10 Sekunden angefeuchtet. Es wurden 8 g einer 30%igen wässrigen Wasserstoffperoxidlösung mit 4 g Basler Blond Claire Blondierpulver vermischt, 2 mL einer 25%igen wässrigen Ammoniaklösung hinzugegeben, erneut vermengt und 8 g der resultierenden Paste mit Hilfe eines Kamms und den geschützten Händen in die Tressenteile einmassiert. Nach einer Einwirkzeit von 30 Minuten bei Raumtemperatur wurden die Tressenteile für 2 Minuten unter laufendem Kranwasser von ca. 35 °C gewaschen und dann mittels eines elektronischen Haartrockners während 3 Minuten unter gleichzeitigem Kämmen getrocknet. Die gesamte Prozedur wurde ausgehend vom Anfeuchten des Tressenteils an einmal wiederholt.

Im Anschluss wurden die Tressenteile entweder mit Haarkonditionierer mit 2% LL-Methionyl-Methionin oder mit Haarkonditionierer mit 2% DD/LL/LD/DL-Methionyl-Methionin (Tabelle 1) in einer Kunststoffschale bedeckt. Die beiden unterschiedlichen Konditioniererbehandlungen wurden jeweils für eine Einwirkzeit von 30 Minuten und von 24 Stunden durchgeführt. Danach wurde unter dem laufenden Wasserstrahl von ca. 35 °C 2 Minuten lang gewaschen und dann mittels eines elektronischen Haartrockners während 3 Minuten unter gleichzeitigem Kämmen getrocknet.

**Tabelle 1. Testformulierungen einfacher Haarkonditionierer. Angaben in Massenprozent. Gewichtsverhältnis DD/LL/LD/DL = 1:1:1:1. Zur Herstellung der Formulierungen wurden dem Fachmann bekannte, übliche Formulierungsverfahren eingesetzt.**

| | Rohstoff | LL-Methionyl-Methionin | DD/LL/LD/DL-Methionyl-Methionin |
|---|---|---|---|
| A | TEGINACID C® (Ceteareth-25) | 0.5% | 0.5% |
| | TEGO Alkanol 1618 (Cetearyl Alcohol) | 2.0% | 2.0% |
| | DD/LL/LD/DL-Methionyl-Methionin | | 2.0% |
| | LL-Methionyl-Methionin | 2.0% | |
| | Wasser | ad 100% | ad 100% |
| | Konservierungsmittel | q.s. | q.s. |
| | Milchsäure (10% in Wasser) | pH 4.0-4.5 | pH 4.0-4.5 |

Die Tressenteile wurden halbiert und ein Teil wurde zur Bestimmung des Methionyl-Methionin-Gehalts der quantitativen Analytik mittels HPLC unterzogen; Der andere Teil wurde zunächst mit Shampoo gewaschen. Für die Behandlung mit Shampoo wurde das Tressenteil nach dem Trocknen unter dem laufenden Wasserhahn angefeuchtet und anschließend 2 mL Shampoo (Tabelle 2) aufgetragen und mit den Händen für ca. 2 Minuten einmassiert. Anschließend wurde das Tressenteil unter dem laufenden Wasserstrahl von ca. 35 °C 2 Minuten lang gewaschen. Das Tressenteil wurde mittels eines elektronischen Haartrockners 3 Minuten lang getrocknet und die Shampoobehandlung viermal wiederholt.

**Tabelle 2. Shampooformulierung. Angaben in Massenprozent. Zur Herstellung der Formulierungen wurden dem Fachmann bekannte, übliche Formulierungsverfahren eingesetzt.**

| | Rohstoff | |
|---|---|---|
| A | Texapon® NSO (Sodium Laureth Sulfate) | 32.10% |
| | REWOMID® C 212 (Cocamide MEA) | 1.75% |
| | TEGO® Betain F 50 (Cocamidopropyl Betaine) | 8.00% |
| | Wasser | ad 100% |
| | Natriumchlorid | 0.20% |
| | Konservierungsmittel | q.s. |
| | Milchsäure (10% in Wasser) | pH 5.5 |

Von den behandelten Tressenteilen (jeweils mit Haarkonditionierer mit 2% LL-Methionyl-Methionin oder mit Haarkonditionierer mit 2% DD/LL/LD/DL-Methionyl-Methionin (Gewichtsverhältnis DD/LL/LD/DL = 1:1:1:1), sowohl mit als auch ohne Shampoobehandlung) wurden jeweils ca. 100-200 mg Haare zur Bestimmung des Methionyl-Methionin-Gehalts entnommen. Die Haarprobe wird in 1-3 mm lange Stücke geschnitten und die Extraktion des Methionyl-Methionins erfolgte im Schraubdeckelgefäß durch zweimalige Extraktion mit einer wässrigen Lösung von 20 mM NaOH (pH -13) unter kräftigem Schütteln während 2 Stunden bei Raumtemperatur. Die Extraktionslösungen wurden vereint und auf 25 mL mit 20 mM NaOH-Lösung aufgefüllt. Davon wurde 1 mL durch Zugabe von ca. 50 µL einer wässrigen Lösung von H₃PO₄ (0.2 mL 85% H₃PO₄ auf 10 mL) neutral bis sauer gestellt und mittels HPLC untersucht.
Die quantitative Analytik erfolgte mittels HPLC-UV auf einem Agilent 1100-System mit Inertsil ODS-3 Säule (250 mm, 4.6 mm ID, 5 µm). Als mobile Phasen wurden verwendet Phase A (870 mL H2O, 5 mL MeCN, 125 mL 0.2 M H3PO4) und Phase B (400 mL H2O, 475 mL MeCN, 125 mL 0.2 M H3PO4) bei 1 mL/min und 30 °C nach Injektion von 20 µL der Analytenlösung. Der Gradient betrug 10% B (0-6 min), dann linear auf 55% B (6-30 min), linear zurück auf 10 % B (30-32 min) und weiter bei 10% B (32-35 min). Die Detektion erfolgte bei 205 nm.
Zur Herstellung einer Kalibrierlösung wurden ca. 20 mg der zu quantifizierenden Referenzsubstanzen in einem 100 mL-Messkolben eingewogen und mit mobiler Phase A bis zur Marke aufgefüllt. Die Lösung wurde ca. 5 Minuten im Ultraschallbad behandelt.

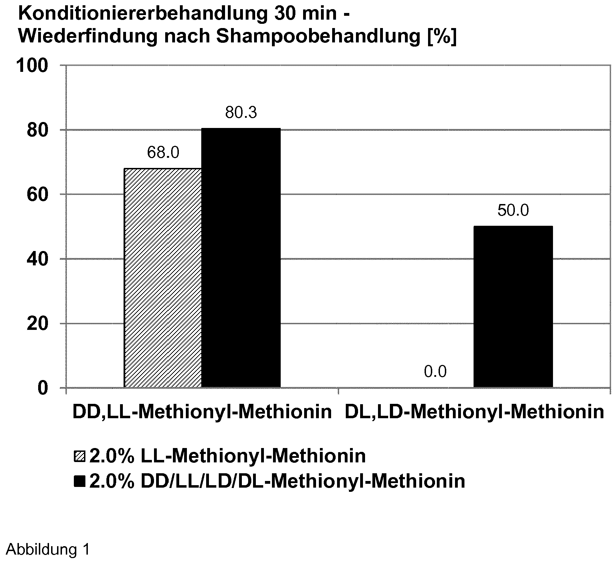

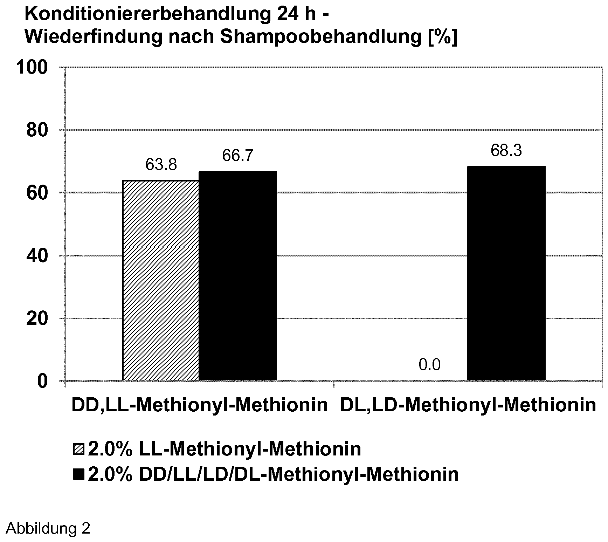

Abbildungen 1 und 2 zeigen jeweils die Resultate, wie sie nach Konditioniererbehandlung für 30 Minuten (Abbildung 1) bzw. 24 Stunden (Abbildung 2) erhalten wurden. Dargestellt ist der verbleibende Anteil an Methionyl-Methionin-Stereoisomeren (in Prozent) am bzw. im Haar nach erfolgter Shampoobehandlung, im Vergleich zu nicht shamponiertem Haar.
Nach Konditioniererbehandlung mit 2% LL-Methionyl-Methionin für 30 Minuten verblieben nach Shampoobehandlung noch 68.0% des DD,LL-Methionyl-Methionin (Summe des Enantiomerenpaares D-Methionyl-D-methionin und L-Methionyl-L-methionin), während dieser Anteil nach Konditioniererbehandlung mit 2% DD/LL/LD/DL-Methionyl-Methionin für 30 Minuten bei 80.3 % lag. Durch Behandlung mit 2% DD/LL/LD/DL-Methionyl-Methionin für 30 Minuten wurden nach Shampoobehandlung zusätzlich noch 50.0% des DL,LD-Methionyl-methionin (Summe des Enantiomerenpaares D-Methionyl-L-methionin und L-Methionyl-D-methionin) wiedergefunden. Nach Konditioniererbehandlung mit LL-Methionyl-Methionin wurde wie erwartet kein DL,LD-Methionyl-methionin nach Extraktion der Haare nachgewiesen.
Ein ähnliches Bild zeigte sich nach Konditioniererbehandlung für 24 Stunden. Im Falle der Behandlung mit 2% LL-Methionyl-Methionin verblieben nach der Shampoobehandlung noch 63.8% des DD,LL-Methionyl-Methionin am bzw. im Haar, während dieser Anteil nach Konditioniererbehandlung mit DD/LL/LD/DL-Methionyl-Methionin bei 66.7% lag. Auch hier wurden nach Shampoobehandlung zusätzlich noch 68.3% des DL,LD-Methionyl-Methionin wiedergefunden.

Die Ergebnisse dieses Versuchs belegen eindrucksvoll die Vorteile einer Haarbehandlung mit DD/LL/LD/DL-Methionyl-Methionin, vor allem im Vergleich zu einer Haarbehandlung mit LL-Methionyl-Methionin. Zum einen wurde gezeigt, dass das besser lösliche Enantiomerenpaar DD/LL-Methionyl-Methionin (21 g/L in Wasser bei Raumtemperatur), ebenso wie das enantiomerenreine LL-Methionyl-Methionin eine Substantivität zum Haar aufweist, da auch nach fünfmaliger Shampoobehandlung noch klar nachweisbare Mengen nach Extraktion der Haare gefunden wurden. Die relativ wiedergefundenen Mengen waren erhöht für den Fall, dass die Haare mit DD/LL/LD/DL-Methionyl-Methionin behandelt wurden, im Vergleich zur Behandlung mit LL-Methionyl-Methionin.
Noch ein weiterer Aspekt belegt ebenfalls den Vorteil einer Haarbehandlung mit DD/LL/LD/DL-Methionyl-Methionin. Obwohl die Löslichkeit des Enantiomerenpaars DL/LD-Methionyl-Methionin bei nur 0.4 g/L liegt, werden überraschenderweise substantielle Mengen davon vom Haar aufgenommen, und die relative Wiederfindung ist auch nach fünfmaliger Shampoobehandlung noch sehr hoch. Im Falle der Konditioniererbehandlung für 24 Stunden ist der relativ wiedergefundene Anteil an DL/LD-Methionyl-Methionin sogar am höchsten, verglichen mit dem relativ wiedergefundenen Anteil an DD/LL-Methionyl-Methionin.
Diese unerwartete, überraschend verbesserte Substantivität zeigt deutlich den Vorteil einer Haarbehandlung mit DD/LL/LD/DL-Methionyl-Methionin, und erklärt die im Folgenden aufgezeigten, hervorragenden Eigenschaften einer Haarbehandlung mit DD/LL/LD/DL-Methionyl-Methionin auf die mechanischen Eigenschaften von geschädigten Haaren.

### Beispiel 2: Verbesserung der mechanischen Eigenschaften von durch Bleichbehandlung geschädigten Haaren durch Behandlung mit Methionyl-Methionin

Ein Tressenteil menschlicher Euro-Natur-Haare, remis, doppelt-gezogen mit einer Länge von 23 cm und einer Breite von 2.0 cm mit einem Gewicht von 2.0 g und dunkelbrauner Farbe wurde unter dem laufenden Wasserhahn innerhalb von 10 Sekunden angefeuchtet. Es wurden 8 g einer 30%igen wässrigen Wasserstoffperoxidlösung mit 4 g Basler Blond Claire Blondierpulver vermischt, 2 mL einer 25%igen wässrigen Ammoniaklösung hinzugegeben, erneut vermengt und 8 g der resultierenden Paste mit Hilfe eines Kamms und den geschützten Händen in das Tressenteil einmassiert. Nach einer Einwirkzeit von 30 Minuten bei Raumtemperatur wurde das Tressenteil für 2 Minuten unter laufendem Kranwasser von ca. 35 °C gewaschen und dann mittels eines elektronischen Haartrockners während 3 Minuten unter gleichzeitigem Kämmen getrocknet. Die gesamte Prozedur wurde ausgehend vom Anfeuchten des Tressenteils an einmal wiederholt.
Im Anschluss wurden je Behandlunsmethode 40 Haare aus dem Tressenteil entfernt und jeweils der mittlere Teil eines Haares mit einer Länge von 3 cm zwischen zwei Messinghülsen mit innenliegender Kunststoffbeschichtung verkrimpt. Die mittlere Fläche jedes einzelnen Haares wurde mittels Dia-stron FDAS760 Faserdimensionalsystem und UvWin PC Applikationssoftware vermessen. Die Haarproben wurden daraufhin in die Probenkassette eines Dia-stron Tensile Tester MTT 670 überführt und jeweils mit mittels Zitronensäure auf pH 7 eingestelltem VE-Wasser versetzt. Eine Messung der einzelnen Haarsträhnen mit der Single Fiber-Methode (Extension 20%, Rate 20 mm/min, Gauge Force 2, Maximum Force 200, Break Threshold 5, Sample Size 30 mm) wurde gestartet. Danach wurden die Haarproben aus der Probenkassette entnommen und in einem Kunststoffschälchen mit VE-Wasser bedeckt.
Für die Behandlung mit Haarkonditionierer wurde nach 30 Minuten das Wasser entfernt und die Haarproben mit Haarkonditionierer bedeckt. Nach einer Einwirkzeit von 30 Minuten wurde jede einzelne Haarprobe unter dem laufenden Wasserstrahl von ca. 35 °C 6 Sekunden lang gewaschen. Die Haarproben wurden über Nacht bei 22 °C und 50% relativer Luftfeuchtigkeit getrocknet.

**Tabelle 3. Testformulierungen einfacher Haarkonditionierer. Angaben in Massenprozent. Gewichtsverhältnis DD/LL/LD/DL = 1:1:1:1. Zur Herstellung der Formulierungen wurden dem Fachmann bekannte, übliche Formulierungsverfahren eingesetzt.**

| | Rohstoff | Vehikel | Wheat Protein | L-Methionin | DD/LL/LD/DL -Methionyl-Methionin |
|---|---|---|---|---|---|
| A | TEGINACID C® (Ceteareth-25) | 0.5% | 0.5% | 0.5% | 0.5% |
| | TEGO Alkanol 1618 (Cetearyl Alcohol) | 2.0% | 2.0% | 2.0% | 2.0% |
| | DD/LL/LD/DL-Methionyl-Methionin | | | | 2.0% |
| | Hydrolyzed Wheat Protein (25% Aktivsubstanz) | | 2.0% | | |
| | L-Methionin | | | 2.0% | |
| | Wasser | ad 100% | ad 100% | ad 100% | ad 100% |
| | Konservierungsmittel | q.s. | q.s. | q.s. | q.s. |
| | Milchsäure (10% in Wasser) | pH 4.0-4.5 | pH 4.0-4.5 | pH 4.0-4.5 | pH 4.0-4.5 |

**Tabelle 4. Testformulierungen erweiterter Haarkonditionierer. Angaben in Massenprozent. Gewichtsverhältnis DD/LL/LD/DL = 35:35:65:65. Zur Herstellung der Formulierungen wurden dem Fachmann bekannte, übliche Formulierungsverfahren eingesetzt.**

| | Rohstoff | Vehikel | Wheat Protein | DD/LL/LD/DL-Methionyl-Methionin |
|---|---|---|---|---|
| A | TEGINACID C® (Ceteareth-25) | 0.5% | 0.5% | 0.5% |
| | TEGO Alkanol 18 (Stearyl Alcohol) | 6.0% | 6.0% | 6.0% |
| | VARISOFT® 300 (Cetrimonium Chloride) DD/LL/LD/DL-Methionyl-Methionin | 6.0% | 6.0% | 6.0% 1.0% |
| | Hydrolyzed Wheat Protein (25% Aktivsubstanz) | | 4.0% | |
| | Wasser | ad 100% | ad 100% | ad 100% |
| | Konservierungsmittel | q.s. | q.s. | q.s. |
| | Milchsäure (10% in Wasser) | pH 4.0 | pH 4.0 | pH 4.0 |

Für die Behandlung mit Shampoo wurde nach 30 Minuten das Wasser entfernt und die Haarproben mit Shampoo bedeckt. Nach einer Einwirkzeit von 5 Minuten wurde jede einzelne Haarprobe unter dem laufenden Wasserstrahl von ca. 35 °C 6 Sekunden lang gewaschen. Die Haarproben wurden mittels eines elektronischen Haartrockners kurz getrocknet und die Shampoobehandlung viermal wiederholt.

**Tabelle 5. Testformulierungen Shampoo. Angaben in Massenprozent. Gewichtsverhältnis DD/LL/LD/DL = 35:35:65:65. Zur Herstellung der Formulierungen wurden dem Fachmann bekannte, übliche Formulierungsverfahren eingesetzt.**

| | Rohstoff | Vehikel | Wheat Protein | DD/LL/LD/DL-Methionyl-Methionin |
|---|---|---|---|---|
| A | Texapon® NSO (Sodium Laureth Sulfate) | 32.10% | 32.10% | 32.10% |
| | REWOMID® C 212 (Cocamide MEA) | 1.75% | 1.75% | 1.75% |
| | TEGO® Betain F 50 (Cocamidopropyl Betaine) | 8.00% | 8.00% | 8.00% |
| | DD/LL/LD/DL-Methionyl-Methionin | | | 1.00% |
| | Hydrolyzed Wheat Protein (25% Aktivsubstanz) | | 4.00% | |
| | Wasser | ad 100% | ad 100% | ad 100% |
| | Natriumchlorid | 0.20% | 0.20% | 0.20% |
| | Konservierungsmittel | q.s. | q.s. | q.s. |
| | Milchsäure (10% in Wasser) | pH 5.5 | pH 5.5 | pH 5.5 |

Im Anschluss an die jeweilige Behandlung wurde die Messung der einzelnen Haarproben beginnend mit der Überführung in die Probenkassette des Dia-stron Tensile Tester MTT 670 wiederholt.
Die Auswertung der Daten erfolgt anhand der Parameter Elastizitätsmodul (E-Modul), Load und/oder Work. Wenn ein Haar gezogen wird, ist die Ausdehnung zunächst proportional zur aufgewendeten Kraft (Hooke'scher Bereich). Das E-Modul beschreibt die aufgewendete Kraft pro Ausdehnung (in Newton/mm²) im Hooke'schen Bereich und repräsentiert somit die Zugfestigkeit einer Haarfaser. Sobald das Haar es bis auf eine Länge von ungefähr 2% gedehnt wurde, verformt es sich sehr schnell weiter bis zu ca. 25-30% seiner ursprünglichen Länge, wobei der Anstieg der dafür benötigten Kraft minimal ist (Yield-Bereich). Der Parameter Load entspricht der Kraft (in Newton), die benötigt wird, um das Haar bis auf 15% zu dehnen und der Parameter Work gibt die benötigte Arbeit zur Dehnung des Haares von 0-15% (in Joule) an.
Zur Messung des Haarermüdungbruchs wurden die Haarproben in die Probenkassette eines Dia-stron Automatic Cyclic Tester (CYC800) überführt und bei 22 °C und 50-55% rel. Luftfeuchte klimatisiert. Die Messung der einzelnen Haarsträhnen erfolgt mit der Constant Stress-Methode (0.015 g/µm², max 100000 cycles, Trigger Load 10 gmf). Die Berechnung der charakteristischen Lebensdauer α einer Haarfaser erfolgt mittels Weibull-Analyse. Der Parameter α repräsentiert die Anzahl der Zyklen, bei der 63.2% aller Haarfasern reissen.

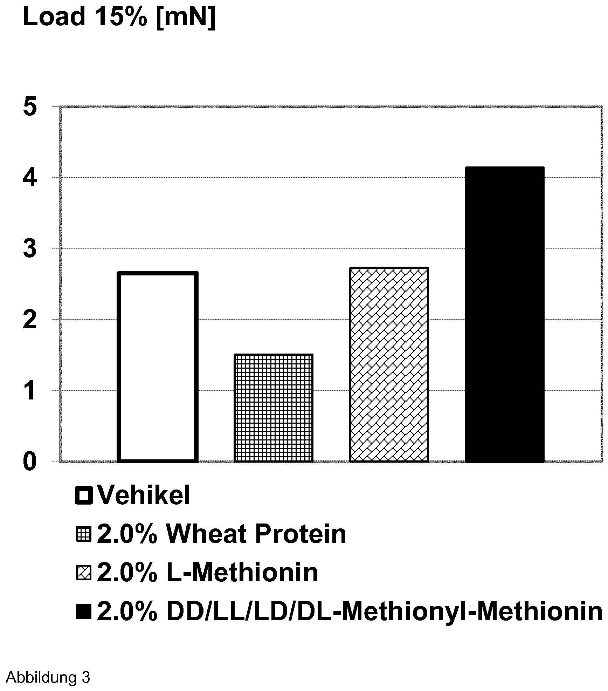

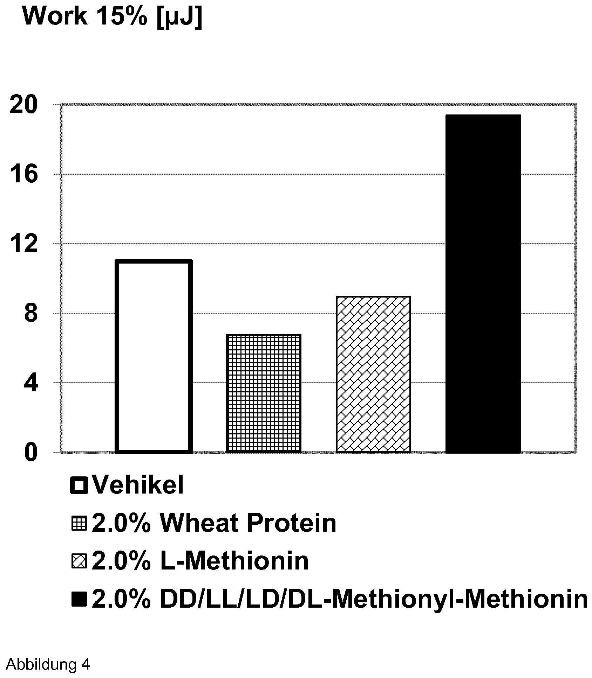

Wie in den Abbildungen 3 und4 zu sehen ist, hatte die Behandlung mit einfachem Haarkonditionierer bereits einen leichten Reparatureffekt auf durch Blondierung geschädigtes Haar, bestimmt durch Messung der Zugdehnungskräfte mittels Tensile Tester, in Höhe von 2.65 mN (Load 15%) bzw. 11.0 µJ (Work 15%). Dieser Reparatureffekt konnte weder durch Zugabe von hydrolysiertem Weizenprotein noch durch Zugabe von L-Methionin zum Haarkonditionierer weiter gesteigert werden. Durch Behandlung mit Haarkonditionierer enthaltend DD/LL/LD/DL-Methionyl-Methionin jedoch konnte der Wert für Load 15% auf 4.14 mN bzw. für Work 15% auf 19.4 µJ erhöht werden. Damit hatte DD/LL/LD/DL-Methionyl-Methionin einen überraschenden und stark ausgeprägten Reparatureffekt auf durch Blondierung geschädigtes Haar.

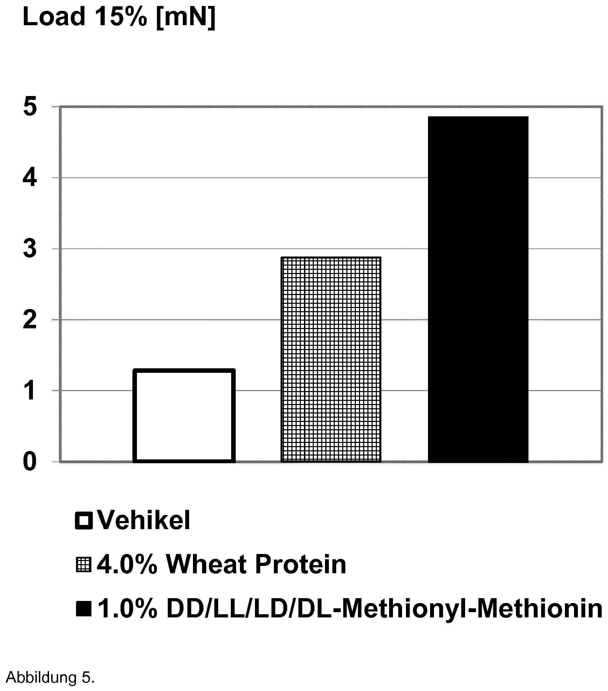

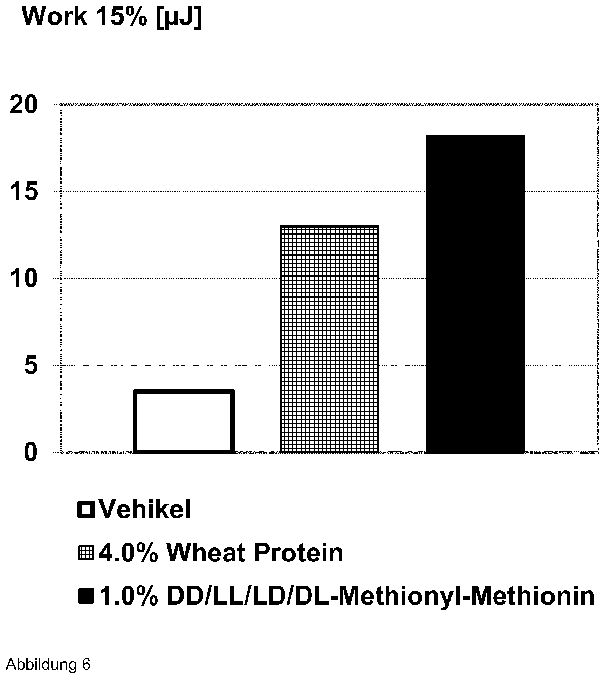

In den Abbildungen 5 und 6 ist der Behandlungseffekt von einem erweiterten Haarkonditionierer auf durch Blondierung geschädigtes Haar zu sehen, bestimmt durch Messung der Zugdehnungskräfte mittels Tensile Tester, in Höhe von 1.28 mN (Load 15%) bzw. 3.51 µJ (Work 15%). Dieser Reparatureffekt konnte durch Zugabe von hydrolysiertem Weizenprotein) zum Haarkonditionierer auf 2.87 mN (Load 15%) und 13.0 µJ (Work 15%) gesteigert werden. Durch Behandlung mit Haarkonditionierer enthaltend DD/LL/LD/DL-Methionyl-Methionin jedoch wurde der Wert für Load 15% noch stärker auf 4.85 mN bzw. für Work 15% auf 18.2 µJ erhöht. Damit hatte Behandlung mit DD/LL/LD/DL-Methionyl-Methionin einen wesentlich stärker ausgeprägten Reparatureffekt auf durch Blondierung geschädigtes Haar als Behandlung mit hydrolysiertem Weizenprotein.

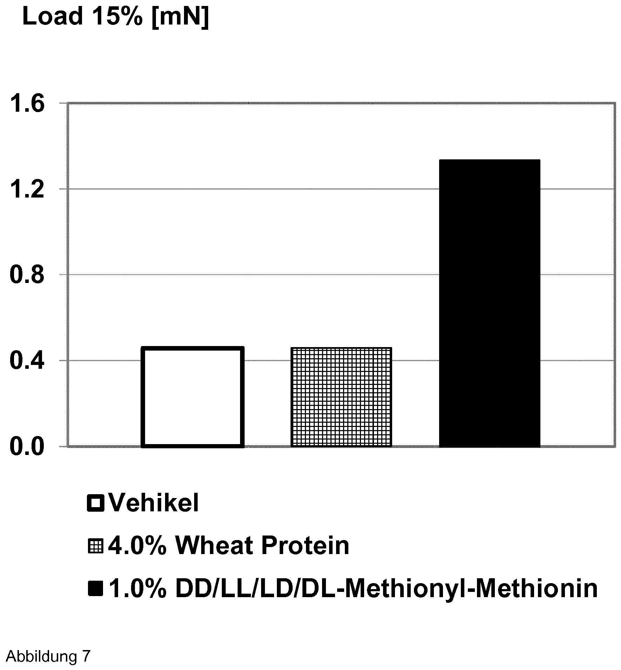

Der Effekt einer Spülung mit Shampoo auf durch Blondierung geschädigtes Haar, bestimmt durch Messung der Zugdehnungskräfte mittels Tensile Tester, ist in Abbildung 7 dargestellt. Der Vehikeleffekt hatte einen Einfluss auf die Zugdehnungskräfte in Höhe von 0.46 mN (Load 15%) bzw. 2.46 µJ (Work 15%). Dieser Reparatureffekt konnte durch Zugabe von hydrolysiertem Weizenprotein zum Haarkonditionierer nicht gesteigert werden, im Falle des Work 15%-Parameters fand sogar eine Reduktion des Reparatureffekts statt. Durch Behandlung mit Haarkonditionierer enthaltend DD/LL/LD/DL-Methionyl-Methionin jedoch wurde der Wert für Load 15% auf 1.33 mN und für Work 15% leicht auf 2.58 µJ erhöht. Damit wurde gezeigt, dass Behandlung von durch Blondierung geschädigtem Haar mit DD/LL/LD/DL-Methionyl-Methionin einen ausgeprägten Reparatureffekt nicht nur aus Leave-In-Anwendungen, sondern ebenfalls aus Rinse-Off-Anwendungen hat. Dieser Reparatureffekt ist bemerkenswert, da die Verweilzeit eines Shampoos auf dem Haar wesentlich kürzer ist als die Verweilzeit eines Leave-In-Haarkonditionierers und konnte nicht durch Behandlung mit marktüblichen Wirkstoffen, wie zum Beispiel hydrolysierten Weizenprotein, erzielt werden.

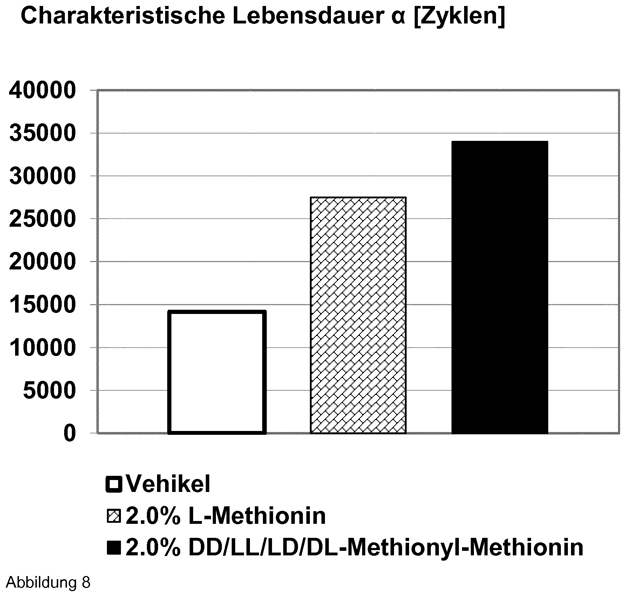

In Abbildung 8 ist die Verbesserung des Haarermüdungsbruchs von durch Blondierung geschädigtem Haar nach Behandlung mit einfachem Haarkonditionierer dargestellt. Die durch Automatic Cyclic Tester gemessenen Zyklen, bei der 63.2% aller Haarfasern reissen, beträgt 14190 nach Vehikelbehandlung. Dieser Wert wurde durch Behandlung mit hydrolysiertem Weizenprotein auf 27490 Zyklen gesteigert, war jedoch noch stärker ausgeprägt nach Behandlung mit DD/LL/LD/DL-Methionyl-Methionin in Höhe von 33980 Zyklen. DD/LL/LD/DL-Methionyl-Methionin führt somit zu einer stärkeren Reduktion des Haarermüdungsbruchs als mit marktgängigen Standards erreicht werden kann.

Zusammenfassend lässt sich sagen, dass der Effekt einer Behandlung mit DD/LL/LD/DL-Methionyl-Methionin auf durch Blondierung geschädigtes Haar bemerkenswert stark ausgeprägt ist, und unerwarteterweise den Effekt einer Behandlung mit marktgängigen Standards weit übertrifft.

### Beispiel 3: Verbesserung der mechanischen Eigenschaften von durch alkalische Glättungsbehandlung geschädigten Haaren durch Behandlung mit Methionyl-Methionin

Ein Tressenteil menschlicher Euro-Natur-Haare, remis, doppelt-gezogen mit einer Länge von 23 cm und einer Breite von 2.0 cm mit einem Gewicht von 2.0 g und dunkelbrauner Farbe wurde unter dem laufenden Wasserhahn innerhalb von 10 Sekunden angefeuchtet. Es wurden 8 g einer Relaxer Cream mit 2 g Relaxer Activator vermischt und die resultierende Paste mit Hilfe eines Pinsels auf das Tressenteil appliziert. Nach einer Einwirkzeit von 15 Minuten bei Raumtemperatur wurde das Tressenteil für 2 Minuten unter laufendem Kranwasser von ca. 35 °C gewaschen und dann mittels eines elektronischen Haartrockners während 3 Minuten unter gleichzeitigem Kämmen getrocknet.

**Tabelle 6. Formulierung Relaxer Cream. Angaben in Massenprozent. Zur Herstellung der Formulierungen wurden dem Fachmann bekannte, übliche Formulierungsverfahren eingesetzt.**

| | Rohstoff | |
|---|---|---|
| A | Wasser | ad 100% |
| | TEGO® Alkanol CS 20 P (Ceteareth-20) | 3.0% |
| B | Petrolatum | 15.0% |
| | Mineral Oil | 10.0% |
| | TEGO® Alkanol 1618 (Cetearyl Alcohol) | 6.0% |
| C | Wasser | 10.0% |
| | Calcium Hydroxide | 5.0% |
| | Propylene Glycol | 2.0% |

**Tabelle 7. Formulierung Relaxer Activator. Angaben in Massenprozent.**

| | Rohstoff | |
|---|---|---|
| | Wasser | ad 100% |
| | Xanthan Gum | 0.2% |
| | Guanidine Carbonate | 25.0% |
| | Propylene Glycol | 2.0% |
| | Konservierungsmittel, Parfüm | q.s. |

Im Anschluss wurden 40 Haare wie in Beispiel 1 beschrieben aus dem Tressenteil entfernt und gemessen. Es erfolgte eine Reparaturbehandlung durch Haarkonditionierer und wiederholte Messung mittels Dia-stron Tensile Tester MTT 670.

**Tabelle 8. Testformulierungen einfacher Haarkonditionierer. Angaben in Massenprozent. Gewichtsverhältnis DD/LL = 1:1. Zur Herstellung der Formulierungen wurden dem Fachmann bekannte, übliche Formulierungsverfahren eingesetzt.**

| | Rohstoff | Vehikel | Wheat Protein | L-Methionin | DD/LL-Methionyl-Methionin |
|---|---|---|---|---|---|
| A | TEGINACID C® (Ceteareth-25) | 0.5% | 0.5% | 0.5% | 0.5% |
| | TEGO Alkanol 1618 (Cetearyl Alcohol) | 2.0% | 2.0% | 2.0% | 2.0% |
| | DD/LL-Methionyl-Methionin | | | | 0.5% |
| | Hydrolyzed Wheat Protein (25% Aktivsubstanz) | | 2.0% | | |
| | L-Methionin | | | 0.5% | |
| | Wasser | ad 100% | ad 100% | ad 100% | ad 100% |
| | Konservierungsmittel | q.s. | q.s. | q.s. | q.s. |
| | Milchsäure (10% in Wasser) | pH 4.0-4.5 | pH 4.0-4.5 | pH 4.0-4.5 | pH 4.0-4.5 |

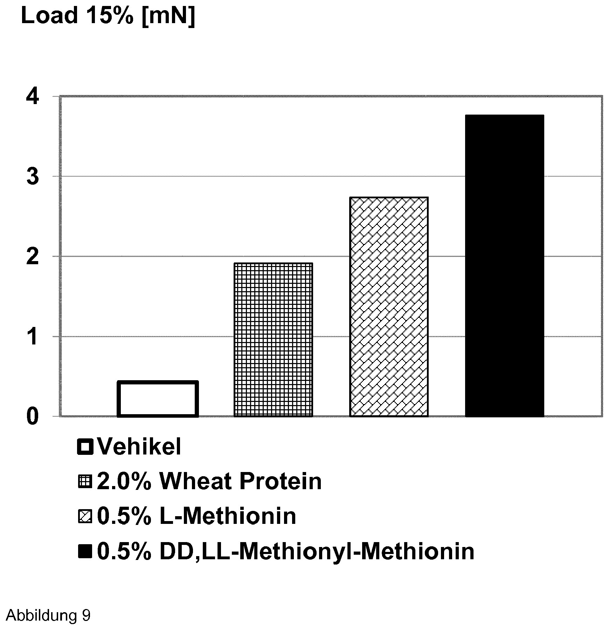

In Abbildung 9 ist der Effekt einer Behandlung mit einfachem Haarkonditionierer auf durch alkalische Glättung geschädigtes Haar, bestimmt durch Messung der Zugdehnungskräfte mittels Tensile Tester, dargestellt. Während Vehikelbehandlung nur einen leichten Reparatureffekt in Höhe von 0.43 mN (Load 15%) ausmachte, konnte der Reparatureffekt durch Zugabe von hydrolysiertem Weizenprotein auf 1.91 mN und durch Zugabe von L-Methionin auf 2.73 mN gesteigert werden. Durch Einsatz von DD/LL-Methionyl-Methionin jedoch konnte der Wert für Load 15% weiter auf 3.76 mN erhöht werden.

Damit hatte DD/LL-Methionyl-Methionin einen überraschenden und stark ausgeprägten Reparatureffekt auf durch alkalische Glättungsbehandlung geschädigtes Haar, der mit marktgängigen Wirkstoffen in dieser Ausprägung nicht erzielt werden kann.

### Beispiel 4: Verbesserung der mechanischen Eigenschaften von durch reduktive Glättungsbehandlung geschädigten Haaren durch Behandlung mit Methionyl-Methionin

Ein Tressenteil menschlicher Euro-Natur-Haare, remis, doppelt-gezogen mit einer Länge von 23 cm und einer Breite von 2.0 cm mit einem Gewicht von 2.0 g und dunkelbrauner Farbe wurde unter dem laufenden Wasserhahn innerhalb von 10 Sekunden angefeuchtet. Anschließend wurden 10 g Basler Haarglättungscreme (für kräftiges Haar) mit Hilfe eines Pinsels auf das Tressenteil appliziert. Während einer Einwirkzeit von 30 Minuten bei Raumtemperatur wird regelmäßig durchgekämmt und anschließend das Tressenteil für 2 Minuten unter laufendem Kranwasser von ca. 35 °C gewaschen. Mittels eines Handtuchs werden die Haare getrocknet, und Basler Cremefixierung (speziell für Haarglättungscreme) mit Hilfe eines Pinsels auf das Tressenteil appliziert. Während einer Einwirkzeit von 10 Minuten bei Raumtemperatur wird regelmäßig durchgekämmt und anschließend das Tressenteil für 2 Minuten unter laufendem Kranwasser von ca. 35 °C gewaschen.
Im Anschluss wurden 40 Haare wie in Beispiel 1 beschrieben aus dem Tressenteil entfernt und gemessen. Es erfolgte eine Reparaturbehandlung durch Haarkonditionierer und wiederholte Messung mittels Dia-stron Tensile Tester MTT 670.

**Tabelle 9. Testformulierungen einfacher Haarkonditionierer. Angaben in Massenprozent. Gewichtsverhältnis DD/LL = 1:1. Zur Herstellung der Formulierungen wurden dem Fachmann bekannte, übliche Formulierungsverfahren eingesetzt.**

| | Rohstoff | Vehikel | Wheat Protein | DD/LL-Methionyl-Methionin |
|---|---|---|---|---|
| A | TEGINACID C® (Ceteareth-25) | 0.5% | 0.5% | 0.5% |
| | TEGO Alkanol 1618 (Cetearyl Alcohol) | 2.0% | 2.0% | 2.0% |
| | DD/LL-Methionyl-Methionin | | | 0.5% |
| | Hydrolyzed Wheat Protein (25% Aktivsubstanz) | | 2.0% | |
| | L-Methionin | | | |
| | Wasser | ad 100% | ad 100% | ad 100% |
| | Konservierungsmittel | q.s. | q.s. | q.s. |
| | Milchsäure (10% in Wasser) | pH 4.0-4.5 | pH 4.0-4.5 | pH 4.0-4.5 |

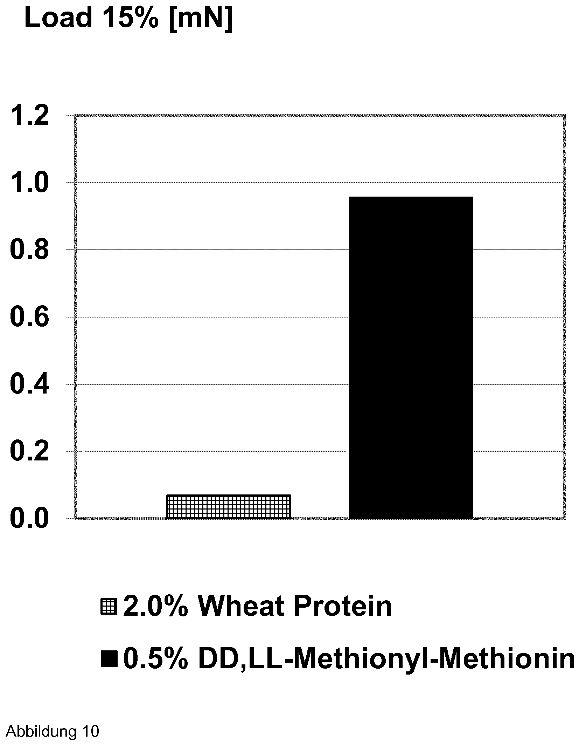

In Abbildung 10 ist der Effekt einer Behandlung mit einfachem Haarkonditionierer auf durch reduktive Glättung geschädigtes Haar, bestimmt durch Messung der Zugdehnungskräfte mittels Tensile Tester, dargestellt. Während Behandlung mit hydrolysiertem Weizenprotein nur einen leichten Reparatureffekt gegenüber Behandlung mit Vehikel in Höhe von 0.07 mN (Load 15%) ausmachte, konnte der Effekt durch Einsatz von DD/LL-Methionyl-Methionin auf 0.96 mN gesteigert werden. Damit hatte DD/LL-Methionyl-Methionin einen überraschenden und stark ausgeprägten Reparatureffekt auf durch redukte Glättungsbehandlung geschädigtes Haar, der mit hydrolysiertem Weizenprotein als marktgängigen Standard nicht erzielt werden kann.

### Beispiel 5: Schutz der Haare vor thermischem Stress durch Behandlung mit Methionyl-Methionin

Ein Tressenteil menschlicher Euro-Natur-Haare, remis, doppelt gezogen mit einer Länge von 23 cm und einer Breite von 2.0 cm mit einem Gewicht von 2.0 g und dunkelbrauner Farbe wurde unter dem laufenden Wasserhahn innerhalb von 10 Sekunden angefeuchtet. Anschließend wurden 2 mL Shampoo (28.6% Texapon NSO in dest. Wasser) für 30 Sekunden in das Haar einmassiert, über weitere 30 Sekunden unter laufendem Kranwasser von ca. 35 °C ausgewaschen, die Haartresse zwischen zwei Fingern ausgestrichen und sechs mal gekämmt. Die Haartresse wird für 1 Minute in 300 mL Wirkstofflösung (Wirkstoff, VE-Wasser, Milchsäure, pH 5.5) eingetaucht, zwischen zwei Fingern ausgestrichen und für 30 Sekunden unter laufendem Kranwasser von ca. 35 °C ausgewaschen und gekämmt. Die Haare wurden mittels eines elektronischen Haartrockners während 3 Minuten unter gleichzeitigem Kämmen getrocknet und anschließend über Nacht in einer Klimakammer (37 °C, 50% rel. Luftfeuchte) gelagert.
Das Tressenteil wird vier mal jeweils über 30 Sekunden durch ein Glätteisen (-210 °C) gezogen, welches mittels eines Gewichts von 630 g auf die Strähne gepresst wird. Diese Behandlung wird ab dem Anfeuchten des Haares und Shampoobehandlung insgesamt fünf mal wiederholt (insgesamt 20 Züge durch das Glätteisen).
Im Anschluss wurden 50 Haare aus dem Tressenteil entfernt und jeweils der mittlere Teil eines Haares mit einer Länge von 3 cm zwischen zwei Messinghülsen mit innen liegender Kunststoffbeschichtung verkrimpt. Die mittlere Fläche jedes einzelnen Haares wurde mittels Dia-stron FDAS760 Faserdimensionalsystem und UvWin PC Applikationssoftware vermessen.
Die Haarproben wurden daraufhin in die Probenkassette eines Dia-stron Automatic Cyclic Tester (CYC800) überführt und bei 22 °C und 50-55% rel. Luftfeuchte klimatisiert. Die Messung der einzelnen Haarsträhnen erfolgt mit der Constant Stress-Methode (0.015 g/µm², maximal 100000 Zyklen, Trigger Load 10 gmf). Die Berechnung der Überlebenswahrscheinlichkeit und der charakteristischen Lebensdauer α einer Haarfaser erfolgt mittels Weibull-Analyse.
Desweiteren werden Haare aus dem Tressenteil mit Hilfe einer Schere zerkleinert und mittels differentieller Scanning-Kalorimetrie (Thermo Instruments Q 1000; Temperaturbereich 50-180 °C; Heizrate 10 °C/min) die Denaturierungstemperatur (T_{D}) bestimmt.

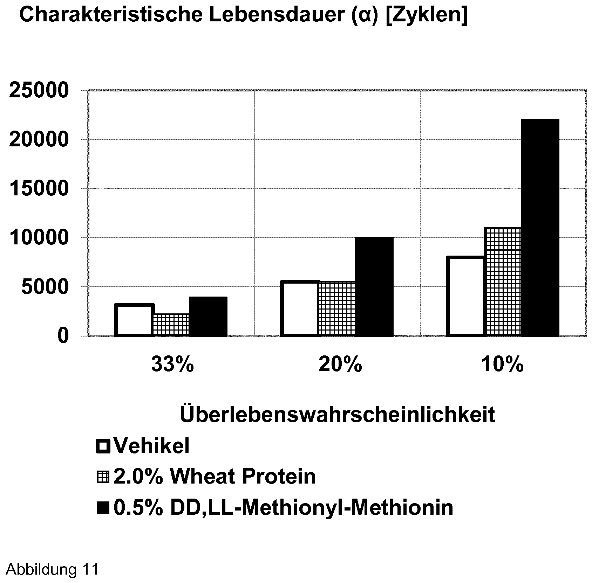

In Abbildung 11 ist der mittels Haarermüdungsbruchmessungen bestimmte Schutz des Haares vor thermischem Stress (Glätteisenbehandlung) durch Vorbehandlung mit Wirkstoff dargestellt. Die charakteristische Lebensdauer α bei unterschiedlichen Überlebenswahrscheinlichkeiten ist angegeben. So beträgt die Anzahl der Zyklen, bei der 20% der Haare intakt bleiben, 5500 nach Schutzbehandlung mit Vehikel. Durch Zugabe von hydrolysiertem Weizenprotein kann dieser Wert nicht gesteigert werden, jedoch waren durch Einsatz von DD/LL-Methionyl-Methionin, Gewichtsverhältnis DD/LL = 1:1, 20% der Haare auch bei 10000 Zyklen noch intakt. Dieser Trend ist ebenfalls bei einer Überlebenswahrscheinlichkeit von 33% zu erkennen. Am deutlichsten wird der Schutzeffekt, wenn die Zahl der Zyklen betrachtet wird, bei der 10% der Haare nicht rissen. So konnte in diesem Fall die Anzahl der Zyklen von 8000 nach Vehikelbehandlung durch Einsatz von hydrolysiertem Weizenprotein leicht auf 11000 erhöht werden, während der Einsatz von DD/LL-Methionyl-Methionin dazu führte, dass auch bei 22000 Zyklen noch 10% der Haare überlebten.

Die Behandlung mit DD/LL-Methionyl-Methionin führt somit zu einer unerwarteten, beeindruckenden Reduktion des Haarermüdungsbruchs, wie sie mit hydrolysierten Weizenprotein als marktgängigen Standard nicht erreicht werden kann.

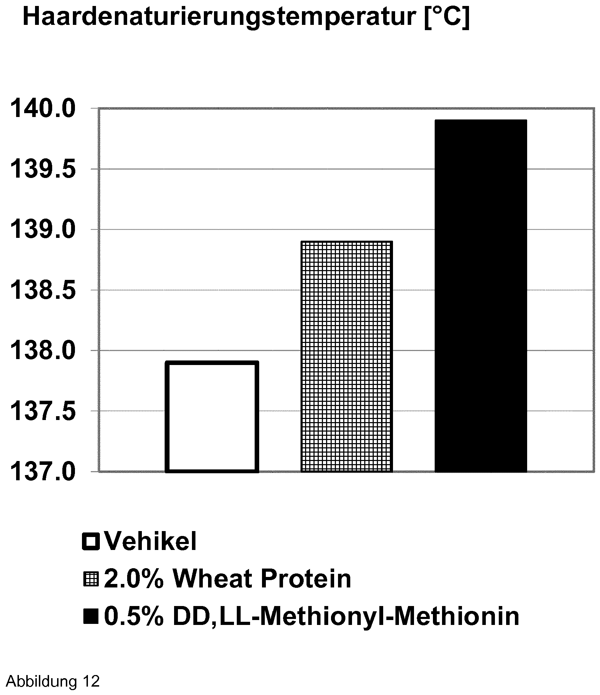

In Abbildung 12 ist der mittels differentieller Scanning-Kalorimetrie bestimmte Schutz des Haares vor thermischem Stress (Glätteisenbehandlung) durch Vorbehandlung mit Wirkstoff dargestellt. Die Denaturierungstemperatur beträgt bei Vehikel-behandelten Haaren 137.9 °C und kann durch Zugabe von hydrolysiertem Weizenprotein) auf 138.9 °C gesteigert werden. Durch Einsatz von DD/LL-Methionyl-Methionin erfolgte eine weitere Erhöhung der Denaturierungstemperatur auf 139.9 °C.
Durch Schutzbehandlung mit DD/LL-Methionyl-Methionin kann somit die Struktur einer Haarfaser stark gekräftigt werden, was durch Behandlung mit einem marktgängigen Standard wie hydrolysierten Weizenprotein nicht in dieser Ausprägung möglich ist.

### Beispiel 6: Haartonikum

| | Rohstoff (INCI) | Anteil |
|---|---|---|
| A | TEGINACID® C (Ceteareth-25) | 3.0% |
| B | Ethanol | 50.0% |
| C | DD/LL-Methionyl-Methionin | 0.7% |
| | Wasser | ad 100% |
| | Milchsäure (10% in Wasser) | pH 5.5 |

### Beispiel 7: Deluxe Haartonikum

| | Rohstoff (INCI) | Anteil |
|---|---|---|
| A | TEGINACID® C (Ceteareth-25) | 3.0% |
| | Sphinganin | 0.2% |
| B | Ethanol | 50.0% |
| C | TEGO® Betain C 60 (Cocamidopropyl Betaine) | 1.0% |
| | HyaCare® (Sodium Hyaluronate) | 0.1% |
| | Koffein | 1.0% |
| | DD/LL/LD/DL-Methionyl-Methionin | 2.0% |
| | Wasser | ad 100% |
| | Milchsäure (10% in Wasser) | pH 5.5 |

### Beispiel 8: Basic Leave-In Haarkonditionierschaum

| | Rohstoff (INCI) | Anteil |
|---|---|---|
| A | ABIL® Quat 3272 (Quaternium-80) | 0.6% |
| | TAGAT® CH 40 (PEG-40 Hydrogenated Castor Oil) | 0.5% |
| | Parfüm | q.s. |
| | TEGO® Betain 810 (Capryl/Capramidopropyl Betaine) | 2.0% |
| | Wasser | ad 100% |
| | DD/LL/LD/DL-Methionyl-Methionin | 1.0% |
| | TEGOCEL® HPM 50 (Hydroxypropyl Methylcellulose) | 0.3% |
| | VARISOFT® 300 (Cetrimonium Chloride) | 1.3% |
| | LACTIL® (Sodium Lactate; Sodium PCA; Glycine; Fructose; Urea; Niacinamide; Inositol; Sodium Benzoate; Lactic Acid) | 0.5% |
| | Zitronensäure, 30% | 0.1% |
| | Konservierungsmittel | q.s. |

### Beispiel 9: Special Clear Leave-In Haarkonditionierschaum

| | Rohstoff (INCI) | Anteil |
|---|---|---|
| A | ABIL® Quat 3272 (Quaternium-80) | 0.6% |
| | TAGAT® CH 40 (PEG-40 Hydrogenated Castor Oil) | 0.5% |
| | Parfüm | q.s. |
| | TEGO® Betain 810 (Capryl/Capramidopropyl Betaine) | 2.0% |
| | Wasser | ad 100% |
| | DD/LL-Methionyl-Methionin | 0.5% |
| | TEGO® Cosmo C 100 (Creatine) | 0.5% |
| | TEGOCEL® HPM 50 (Hydroxypropyl Methylcellulose) | 0.3% |
| | VARISOFT® 300 (Cetrimonium Chloride) | 1.3% |
| | TEGO® Natural Betaine (Betaine) | 0.5% |
| | Zitronensäure, 30% | 0.1% |
| | Konservierungsmittel | q.s. |

### Beispiel 10: Hair repair Leave-In Haarkonditionier-Spray

| | Rohstoff (INCI) | Anteil |
|---|---|---|
| A | TAGAT® CH 40 (PEG-40 Hydrogenated Castor Oil) | 2.00% |
| | Ceramide III (Ceramide NP) | 0.05% |
| | Parfüm | q.s. |
| | Wasser | ad 100% |
| | ABIL® Quat 3272 (Quaternium-80) | 0.50% |
| | ABIL® B 88183 (PEG/PPG-20 / 6 Dimethicone) | 1.50% |
| | TEGO® Smooth (Betaine; Urea; Potassium Lactate; Sodium Polyglutamate; Hydrolyzed Sclerotium Gum) | 2.00% |
| | DD/LL/LD/DL-Methionyl-Methionin | 3.00% |
| | TEGO® Betain F 50 (Cocamidopropyl Betaine) | 2.00% |
| | Zitronensäure, (10% in Wasser) | 2.00% |
| | Konservierungsmittel | q.s. |

### Beispiel 11: Leave-in Haarkonditionier-Mousse

| | Rohstoff (INCI) | Anteil |
|---|---|---|
| A | ABIL® Quat 3272 (Quaternium-80) | 0.5% |
| | ABIL® B 88183 (PEG/PPG-20 / 6 Dimethicone) | 0.4% |
| | TAGAT® CH 40 (PEG-40 Hydrogenated Castor Oil) | 0.5% |
| | Parfüm | q.s. |
| | TEGO® Betain 810 (Capryl/Capramidopropyl Betaine) | 4.0% |
| | Wasser | ad 100% |
| | Panthenol | 0.2% |
| | TEGO® Natural Betaine (Betaine) | 0.3% |
| | DD/LL/LD/DL-Methionyl-Methionin | 1.0% |
| | Zitronensäure (30% in Wasser) | 0.4% |
| | Konservierungsmittel | q.s. |

### Beispiel 12: Repair Leave-In Haarkonditionierer

| | Rohstoff (INCI) | Anteil |
|---|---|---|
| A | TEGINACID® C (Ceteareth-25) | 4.0% |
| | Cyclopentasiloxane, Dimethiconol | 20.0% |
| | ABIL® Soft AF 100 (Methoxy PEG/PPG-7/3 Aminopropyl Dimethicone) | 1.0% |
| | TEGO® Alkanol L 4 (Laureth-4) | 0.5% |
| B | TEGO® Carbomer 140 G (Carbomer) | 0.5% |
| | Wasser | ad 100% |
| | 1.2-Propylenglykol (Propylene Glycol) | 5.0% |
| | DD/LL/LD/DL-Methionyl-Methionin | 4.0% |
| C | Natriumhydroxid | 0.2% |
| | Konservierungsmittel, Parfüm | q.s. |

### Beispiel 13: Leave-in Haarkonditionierer mit Spliss-Repair

| | Rohstoff (INCI) | Anteil |
|---|---|---|
| A | TEGINACID® C (Ceteareth-25) | 4.0% |
| | Cyclopentasiloxane, Dimethiconol | 20.0% |
| | ABIL® Soft AF 100 (Methoxy PEG/PPG-7/3 Aminopropyl Dimethicone) | 1.0% |
| | TEGO® Alkanol L 4 (Laureth-4) | 0.5% |
| | VARISOFT® BT 85 Pellets (Behentrimonium Chloride) | 0.4% |
| B | Wasser | ad 100% |
| | DD/LL/LD/DL-Methionyl-Methionin | 3.0% |
| | Propylene Glycol | 5.0% |
| | TEGO® Carbomer 340 FD (Carbomer) | 0.5% |
| C | Natriumhydroxid (25% in Wasser) | ad pH 5-6 |
| | Konservierungsmittel, Parfüm | q.s. |

### Beispiel 14: Style & Relax Leave-in Haarkonditionierer

| | Rohstoff (INCI) | Anteil |
|---|---|---|
| A | ABIL® EM 90 (Cetyl PEG/PPG-10/1 Dimethicone) | 2.0% |
| | Paraffinum Perliquidum | 2.0% |
| | Vaseline | 2.0% |
| | ABIL® Wax 9800 (Stearyl Dimethicone) | 2.0% |
| | Avocado (Persea Gratissima) Oil | 2.0% |
| | Olive (Olea Europaea) Oil | 2.0% |
| | TEGOSOFT® OP (Ethylhexyl Palmitate) | 3.0% |
| | TEGOSOFT® P (Isopropyl Palmitate) | 3.0% |
| | Simmondsia Chinensis (Jojoba) Seed Oil | 2.0% |
| | Tocopheryl Acetate | 0.2% |
| B | ABIL® Soft AF 100 (Methoxy PEG/PPG-7/3 Aminopropyl Dimethicone) | 0.5% |
| | Glycerin | 3.0% |
| | Natriumchlorid | 0.7% |
| | Wasser | ad 100% |
| | DD/LL-Methionyl-Methionin | 1.5% |
| C | Konservierungsmittel, Parfüm | q.s. |

### Beispiel 15: "HPP" Clear Leave-in Haarkonditionierschaum

| | Rohstoff (INCI) | Anteil |
|---|---|---|
| A | ABIL® T Quat 60 (Silicone Quaternium-22) | 0.5% |
| | TAGAT® CH 40 (PEG-40 Hydrogenated Castor Oil) | 0.5% |
| | Parfüm | q.s. |
| | TEGO® Betain 810 (Capryl/Capramidopropyl Betaine) | 2.0% |
| | Wasser | ad 100% |
| | DD/LL-Methionyl-Methionin | 0.5% |
| | TEGO® Cosmo C 100 (Creatine) | 0.5% |
| | TEGOCEL® HPM 50 (Hydroxypropyl Methylcellulose) | 0.3% |
| | VARISOFT® 300 (Cetrimonium Chloride) | 1.3% |
| | LACTIL® (Sodium Lactate; Sodium PCA; Glycine; Fructose; Urea; Niacinamide; Inositol; Sodium Benzoate; Lactic Acid) | 0.5% |
| | Zitronensäure, 30% | 0.1% |
| | Konservierungsmittel | q.s. |

### Beispiel 16: Rich and creamy Haarkonditionierer

| | Rohstoff (INCI) | Anteil |
|---|---|---|
| A | Wasser | ad 100% |
| | Glycerin | 2.00% |
| | Propylene Glycol | 2.00% |
| | DD/LL/LD/DL-Methionyl-Methionin | 2.50% |
| | VARISOFT® PATC (Palmitamidopropyltrimonium Chloride) | 2.00% |
| | TEGOSOFT® liquid (Cetearyl Ethylhexanoate) | 2.00% |
| | TEGOSOFT® HP (Isocetyl Palmitate) | 1.00% |
| | REWODERM® LI S 80 (PEG-200 Hydrogenated Glyceryl Palmate; PEG-7 Glyceryl Cocoate) | 0.50% |
| | TEGO® Alkanol 1618 (Cetearyl Alcohol) | 4.50% |
| | TEGOSOFT® MM (Myristyl Myristate) | 1.50% |
| | ABIL® B 8852 (PEG/PPG-4/12 Dimethicone) | 0.50% |
| | Tocopheryl Acetate | 0.25% |
| | Panthenol | 0.25% |
| | Wasser, Konservierungsmittel | q.s. |

### Beispiel 17: Brilliantine Cream für kurzes Haar

| | Rohstoff (INCI) | Anteil |
|---|---|---|
| A | ABIL® EM 90 (Cetyl PEG/PPG-10/1 Dimethicone) | 2.5% |
| | Paraffinum Perliquidum | 7.0% |
| | Lunacera MWN (Microwax) | 1.5% |
| | Hydrogenated Castor Oil | 0.5% |
| | TEGOSOFT® CT (Caprylic/Capric Triglyceride) | 6.0% |
| | Prunus Amygdalus Dulcis Oil | 0.5% |
| B | Wasser | ad 100% |
| | DD/LL-Methionyl-Methionin | 0.5% |
| | Natriumchlorid | 0.5% |
| | Glycerin | 3.0% |

### Beispiel 18: Basic Hair and Body Shampoo

| | Rohstoff (INCI) | Anteil |
|---|---|---|
| A | REWOTERIC® AM C (Sodium Cocoamphoacetate) | 15.00% |
| | REWOPOL® SB F 12 P (Disodium Lauryl Sulfosuccinate) | 3.80% |
| | ANTIL® Soft SC (Sorbitan Sesquicaprylate) | 0.90% |
| | Parfüm | q.s. |
| | Wasser | ad 100% |
| | DD/LL/LD/DL-Methionyl-Methionin | 0.70% |
| | TEGO® Betain F 50 (Cocamidopropyl Betaine) | 13.00% |
| | Zitronensäure (30% in Wasser) | 3.00% |
| | Konservierungsmittel | q.s. |

### Beispiel 19: Haarkonditionier-Shampoo PEG- and sulfate free

| | Rohstoff (INCI) | Anteil |
|---|---|---|
| A | REWOTERIC® AM C (Sodium Cocoamphoacetate) | 15.0% |
| | REWOPOL® SB F 12 P (Disodium Lauryl Sulfosuccinate) | 3.8% |
| | Wasser | ad 100% |
| | DD/LL/LD/DL-Methionyl-Methionin | 1.5% |
| | TEGO® Betain F 50 (Cocamidopropyl Betaine) | 10.0% |
| | VARISOFT® PATC (Palmitamidopropyltrimonium Chloride) | 2.3% |
| | REWOMID® SPA (Isostearamide MIPA) | 1.0% |

### Beispiel 20: Clear Hair Shampoo mit UV-Schutzeigenschaften

| | Rohstoff (INCI) | Anteil |
|---|---|---|
| A | VARISOFT® PATC (Palmitamidopropyltrimonium Chloride) | 1.0% |
| | ABIL® UV Quat 50 (Polysilicone-19) | 1.0% |
| | Parfüm | q.s. |
| | Wasser | ad 100% |
| | DD/LL-Methionyl-Methionin | 0.5% |
| | Sodium Laureth Sulfate, 28% | 32.0% |
| | TEGO® Betain F 50 (Cocamidopropyl Betaine) | 8.0% |
| | ANTIL® 171 (PEG-18 Glyceryl Oleate/Cocoate) | 2.0% |
| | Konservierungsmittel | q.s. |

### Beispiel 21: Pearl & Protect Haarkonditionier-Shampoo

| | Rohstoff (INCI) | Anteil |
|---|---|---|
| A | Wasser | ad 100% |
| | DD/LL/LD/DL-Methionyl-Methionin | 3.00% |
| | Polyquaternium-10 | 0.20% |
| | ABIL® T Quat 60 (Silicone Quaternium-22) | 0.80% |
| | Sodium Laureth Sulfate, 28% | 25.00% |
| | REWOTERIC® AM C (Sodium Cocoamphoacetate) | 8.00% |
| | REWOPOL® SB C 55 (Disodium PEG-5 Laurylcitrate Sulfosuccinate; Capryl/Capramidopropyl Betaine) | 4.60% |
| | Zitronensäure (30% in Wasser) | 1.80% |
| | ANTIL® 120 | 0.20% |
| | VARISOFT® PATC (Palmitamidopropyltrimonium Chloride) | 1.00% |
| | TEGO® Pearl N 300 (Glycol Distearate; Laureth-4; Cocamidopropyl Betaine) | 2.00% |
| | Konservierungsmittel, Parfüm | q.s. |

### Beispiel 22: Clear Haarkonditionier-Shampoo mit Ceramid

| | Rohstoff (INCI) | Anteil |
|---|---|---|
| A | Ceramide IIIB (Ceramide NP) | 0.05% |
| | Sodium Laureth Sulfate, 28% | 30.00% |
| | Parfüm | q.s. |
| | ABIL® Quat 3272 (Quaternium-80) | 0.50% |
| | Wasser | ad 100% |
| | DD/LL-Methionyl-Methionin | 0.50% |
| | TEGO® Betain F 50 (Cocamidopropyl Betaine) | 10.00% |
| | ANTIL® 171 (PEG-18 Glyceryl Oleate/Cocoate) | 2.00% |
| | Natriumchlorid | q.s. |
| | Konservierungsmittel | q.s. |

### Beispiel 23: Haarkonditionier-Anti-Schuppen Shampoo

| | Rohstoff (INCI) | Anteil |
|---|---|---|
| A | TEGIN® G 1100 Pellets (Glycol Distearate) | 3.0% |
| | Sodium Laureth Sulfate, 28% | 40.0% |
| B | Parfüm | q.s. |
| | Zinc-Pyrion NF (48%) (Zinc Pyrithione) | 2.0% |
| | ABIL® Quat 3272 (Quaternium-80) | 1.0% |
| C | Wasser | ad 100% |
| | DD/LL/LD/DL-Methionyl-Methionin | 2.5% |
| | TEGO® Carbomer 341 ER (Acrylates/C10-30 Alkyl Acrylate Crosspolymer) | 0.2% |
| | Polyquaternium-10 | 0.3% |
| | Natriumhydroxid, 25% in Wasser | 0.3% |
| D | REWOTERIC® AM B U 185 (Undecylenamidopropyl Betaine) | 12.5% |
| | ANTIL® SPA 80 (Isostearamide MIPA; Glyceryl Laurate) | 3.7% |
| Z | Konservierungsmittel, Parfüm | q.s. |

### Beispiel 24: 2in1 Shampoo

| | Rohstoff (INCI) | Anteil |
|---|---|---|
| A | Sodium Laureth Sulfate, 28% | 20.0% |
| | REWOPOL® SB FA 30 B (Disodium Laureth Sulfosuccinate) | 6.0% |
| | TEGOSOFT® LSE 65 K SOFT (Sucrose Cocoate) | 2.5% |
| | ABIL® Quat 3272 (Quaternium-80) | 2.0% |
| | Wasser | ad 100% |
| | DD/LL/LD/DL-Methionyl-Methionin | 3.5% |
| | Ucare Polymer JR 400 (Polyquaternium-10) | 0.1% |
| | TEGO® Betain F 50 (Cocamidopropyl Betaine) | 7.0% |
| | VARISOFT® PATC (Palmitamidopropyltrimonium Chloride) | 2.5% |
| | REWODERM® LI S 80 (PEG-200 Hydrogenated Glyceryl Palmate; PEG-7 Glyceryl Cocoate) | 3.0% |
| | TEGO® Pearl N 100 (Glycol Distearate; Steareth-4) | 2.0% |
| | Konservierungsmittel, Parfüm | q.s. |

### Beispiel 25: Haarkonditionier-Gel

| | Rohstoff (INCI) | Anteil |
|---|---|---|
| A | PEG-20 Glyceryl Laurate | 3.00% |
| | Parfüm | q.s. |
| | ABIL® B 88183 (PEG/PPG-20 / 6 Dimethicone) | 2.00% |
| B | Wasser | ad 100% |
| | DD/LL-Methionyl-Methionin | 1.50% |
| | TEGO® Carbomer 140 G (Carbomer) | 1.50% |
| C | Natriumhydroxid (25% in Wasser) | 2.20% |
| | Konservierungsmittel | q.s. |

### Beispiel 26: Sonnenschutz Ringing Gel Wax mit Mica

| | Rohstoff (INCI) | Anteil |
|---|---|---|
| A | Wasser | ad 100% |
| | Propylene Glycol | 2.00% |
| | Glycerin | 11.00% |
| | DD/LL-Methionyl-Methionin | 1.00% |
| | ABIL® Soft AF 100 (Methoxy PEG/PPG-7/3 Aminopropyl Dimethicone) | 0.50% |
| B | TEGO® Alkanol IC 20 (Isoceteth-20) | 14.50% |
| | ABIL® UV Quat 50 (Polysilicone-19) | 2.00% |
| | TEGO® Carbomer 140 G (Carbomer) | 1.50% |
| | TEGO® Alkanol L 4 (Laureth-4) | 10.00% |
| | Paraffinum Perliquidum | 6.00% |
| | TEGOSOFT® TN (C12-15 Alkyl Benzoate) | 6.00% |
| | Timiron Splendid Gold (Titanium Dioxide; Mica; Silicia) | 0.05% |
| C | Parfüm | q.s. |

### Beispiel 27: Haarkonditionier-Rinse mit UV-Schutzeigenschaften, PEG-free

| | Rohstoff (INCI) | Anteil |
|---|---|---|
| A | TEGO® Alkanol 1618 (Cetearyl Alcohol) | 5.0% |
| | ABIL® UV Quat 50 (Polysilicone-19) | 2.0% |
| B | Wasser | ad 100% |
| | Glycerin | 2.0% |
| | DD/LL/LD/DL-Methionyl-Methionin | 3.0% |
| | VARISOFT® BT 85 Pellets (Behentrimonium Chloride) | 1.0% |
| C | Konservierungsmittel, Parfüm | q.s. |

### Beispiel 28: Combi-Mix Clear Haarkonditionier-Shampoo

| | Rohstoff (INCI) | Anteil |
|---|---|---|
| A | Sodium Laureth Sulfate, 28% | 28.0% |
| | ABIL® Soft AF 100 (Methoxy PEG/PPG-7/3 Aminopropyl Dimethicone) | 0.4% |
| | Parfüm | q.s. |
| | Wasser | ad 100% |
| | DD/LL-Methionyl-Methionin | 0.7% |
| | TEGO® Cosmo C 100 (Creatine) | 1.0% |
| | TEGO® Betain F 50 (Cocamidopropyl Betaine) | 11.0% |
| | ANTIL® 171 (PEG-18 Glyceryl Oleate/Cocoate) | 2.0% |
| | Natriumchlorid | q.s. |
| | Konservierungsmittel | q.s. |

### Beispiel 29: Repair and Pearl Haarkonditionier-Shampoo

| | Rohstoff (INCI) | Anteil |
|---|---|---|
| A | Wasser | ad 100% |
| | DD/LL/LD/DL-Methionyl-Methionin | 2.00% |
| | ABIL® Quat 3272 (Quaternium-80) | 0.60% |
| | Sodium Laureth Sulfate, 28% | 30.00% |
| | REWOTERIC® AM C (Sodium Cocoamphoacetate) | 8.00% |
| | REWOPOL® SB CS 50 B (Disodium PEG-5 Laurylcitrate Sulfosuccinate; Sodium Laureth Sulfate) | 6.00% |
| | Zitronensäure (30% in Wasser) | 1.80% |
| | ANTIL® 120 PLUS (PEG-120 Methyl Glucose Dioleate) | 0.20% |
| | VARISOFT® PATC (Palmitamidopropyltrimonium Chloride) | 2.50% |
| | Natriumchlorid | 0.40% |
| | TEGO® Pearl N 300 (Glycol Distearate; Laureth-4; Cocamidopropyl Betaine) | 2.00% |
| | Konservierungsmittel | q.s. |

## Patentansprüche

1. Kosmetische Formulierung enthaltend mindestens ein Methionyl-Methionin-Stereoisomer ausgewählt aus der Gruppe
D-Methionyl-L-Methionin, L-Methionyl-D-Methionin und D-Methionyl-D-Methionin, **dadurch gekennzeichnet, dass** sie ein Gewichtverhältnis von DL- und LD-Methionyl-Methionin zu DD- und LL-Methionyl-Methionin von 9 zu 1 bis 3 zu 2 aufweist.

2. Kosmetische Formulierung gemäß Anspruch 1 zusätzlich enthaltend L-Methionyl-L-Methionin.

3. Verwendung von Methionyl-Methionin zur Konditionierung von Haaren.

4. Verwendung von Methionyl-Methionin zur Erhöhung der Zugfestigkeit der Haare.

5. Verwendung von Methionyl-Methionin zur Erhöhung der Belastbarkeit von Haaren.

6. Verwendung von Methionyl-Methionin zum Schutz der Haare vor oxidativer Schädigung, zum Schutz der Haare vor thermischer Schädigung und/oder zum Schutz der Haare vor chemischer Schädigung

7. Verwendung von Methionyl-Methionin zur Verbesserung der mechanischen Eigenschaften von Nägeln.

8. Verwendung gemäß einem der Ansprüche 3 bis 7, **dadurch gekennzeichnet, dass** eine Formulierung gemäß einem der Ansprüche 1 oder 2 verwendet wird.

## Claims

1. Cosmetic formulation comprising at least one methionyl-methionine stereoisomer selected from the group D-methionyl-L-methionine, L-methionyl-D-methionine and D-methionyl-D-methionine, **characterized in that** it has a weight ratio of DL- and LD-methionyl-methionine to DD- and LL-methionyl-methionine of 9:1 to 3:2.

2. Cosmetic formulation according to Claim 1, additionally comprising
L-methionyl-L-methionine.

3. Use of methionyl-methionine for conditioning hair.

4. Use of methionyl-methionine for increasing the tensile strength of the hair.

5. Use of methionyl-methionine for increasing the loadability of hair.

6. Use of methionyl-methionine for protecting the hair against oxidative damage, for protecting the hair against thermal damage and/or for protecting the hair against chemical damage.

7. Use of methionyl-methionine for improving the mechanical properties of nails.

8. Use according to one of Claims 3 to 7, **characterized in that** a formulation according to one of Claims 1 and 2 is used.

## Revendications

1. Formulation cosmétique contenant au moins un stéréoisomère de méthionyl-méthionine choisi dans le groupe constitué par la D-méthionyl-L-méthionine, la L-méthionyl-D-méthionine et la D-méthionyl-D-méthionine, **caractérisée en ce qu'**elle présente un rapport en poids entre la DL- et LD-méthionyl-méthionine et la DD- et LL-méthionyl-méthionine de 9 sur 1 à 3 sur 2.

2. Formulation cosmétique selon la revendication 1, contenant en outre de la L-méthionyl-L-méthionine.

3. Utilisation de méthionyl-méthionine pour le conditionnement de cheveux.

4. Utilisation de méthionyl-méthionine pour augmenter la résistance à la traction de cheveux.

5. Utilisation de méthionyl-méthionine pour augmenter la capacité de charge de cheveux.

6. Utilisation de méthionyl-méthionine pour protéger des cheveux d'un endommagement oxydatif, pour protéger des cheveux d'un endommagement thermique et/ou pour protéger des cheveux d'un endommagement chimique.

7. Utilisation de méthionyl-méthionine pour améliorer les propriétés mécaniques d'ongles.

8. Utilisation selon l'une quelconque des revendications 3 à 7, **caractérisée en ce qu'**une formulation selon l'une quelconque des revendications 1 ou 2 est utilisée.
